(19) Europäisches Patentamt / European Patent Office / Office européen des brevets

(11) **EP 1 570 744 B1**

(12) **EUROPEAN PATENT SPECIFICATION**

(45) Date of publication and mention
of the grant of the patent:
**03.09.2014 Bulletin 2014/36**

(51) Int Cl.:
*A23C 19/08* (2006.01)          *A23C 9/13* (2006.01)
*A23L 1/30* (2006.01)          *A23J 3/34* (2006.01)

(21) Application number: **03774166.7**

(22) Date of filing: **21.11.2003**

(86) International application number:
**PCT/JP2003/014954**

(87) International publication number:
**WO 2004/047543 (10.06.2004 Gazette 2004/24)**

(54) **FUNCTIONAL FOODS AND PROCESS FOR PRODUCING FUNCTIONAL FOOD**

FUNCTIONAL FOODS UND VERFAHREN ZUR HERSTELLUNG VON FUNCTIONAL FOOD

PRODUITS ALIMENTAIRES FONCTIONNELS ET LEUR PROCEDE DE PREPARATION

(84) Designated Contracting States:
**AT BE BG CH CY CZ DE DK EE ES FI FR GB GR HU IE IT LI LU MC NL PT RO SE SI SK TR**

(30) Priority: **28.11.2002 JP 2002346408**

(43) Date of publication of application:
**07.09.2005 Bulletin 2005/36**

(73) Proprietor: **Meiji Co., Ltd.**
**Tokyo (JP)**

(72) Inventors:
• **SUZUKI, Masayuki,**
  **Meiji Dairies Corporation**
  **Odawara-shi, Kanagawa 250-0862 (JP)**
• **TONOUCHI, Hidekazu,**
  **Meiji Dairies Corporation**
  **Odawara-shi,**
  **Kanagawa 250-0862 (JP)**
• **YOSHIOKA, Norimichi,**
  **Meiji Dairies Corporation**
  **Oadawara-shi,**
  **Kanagawa 250-0862 (JP)**
• **UCHIDA, Masayuki,**
  **Meiji Dairies Corporation**
  **Odawara-shi,**
  **Kanagawa 250-0862 (JP)**

• **ODA, Munehiro,**
  **Meiji Dairies Corporation**
  **Odawara-shi,**
  **Kanagawa 250-0862 (JP)**

(74) Representative: **Isarpatent**
**Patent- und Rechtsanwälte**
**Postfach 44 01 51**
**80750 München (DE)**

(56) References cited:
**EP-A1- 0 469 857     JP-A- 3 160 944**
**JP-A- 4 179 441**

• **MEISEL H. ET AL: 'ACE-inhibitory activities in milk products' MILCHWISSENSCHAFT vol. 52, no. 6, 1997, pages 307 - 311, XP002986735**
• **RYHANEN EEVA-LIISA ET AL: 'A new type of ripened low-fat cheese with bioactive properties' INTERNATIONAL DAILY JOURNAL vol. 11, no. 4/7, 2001, pages 441 - 447, XP002986736**
• **TADASHI ITO: 'Cheese-chu no Angiotensin Henkan Koso Inhibitor' MEDICINE AND BIOLOGY vol. 115, no. 6, 10 December 1987, pages 375 - 377, XP002977491**

EP 1 570 744 B1

**Description**

Field of the Invention

This invention relates to a process for producing functional food, more specifically a process for producing functional food of which angiotensin converting enzyme inhibitory activity is enhanced.

Background of Invention

High blood pressure is a risk factor for cerebral stroke and heart disease and is remedied by diet therapy, life style improvement and moreover, drug therapy by administering hypotensive drugs. However, since drug therapy always produces side effects, it is better not to rely on drug therapy. On the other hand, it has recently been reported by T. Saito et al. (Isolation and Structural Analysis of Antihypertensive Peptides That Exist Naturally in Gouda Cheese, Journal of Dairy Science, USA, Vol. 83, No. 7, 2000, p. 1434-1440), H. Meisel et al. (ACE-inhibitory activities in milk products, Milchwissenschaft, Germany, 52 (6), 1997, p. 307-311), and T. Ito et al. (Angiotensin converting enzyme inhibitor in cheese, Medicine and Biology, Vol. 115, No. 6, 1987, p. 375-377) that peptides existing in food contains hypotensive peptides that have the effect of preventing high blood pressure and maintaining blood pressure within the normal range. The mechanism is that the abovementioned hypotensive peptides inhibit the functions of angiotensin converting enzyme (ACE). ACE is an enzyme that converts angiotensin I, a peptide in blood, to angiotensin II with a pressor function, inactivates kinin with a hypotensive function, and promotes a pressor effect. Furthermore, beverages that contain hypotensive peptides existing in food and have an excellent antihypertensive effect are commercially available, for example, as a trade name Ameel S manufactured by Calpis Co., Ltd.

Hypotensive peptides are also present in cheese. Animal tests using rats have proved that cheese fed to them prevents high blood pressure. It can be said that, if eating cheese daily is effective, then high blood pressure can be prevented more naturally than by ingesting beverage containing hypotensive peptides. Thus, the inventors have measured the ACE inhibitory activity of processed cheeses, which are commercially available, to obtain the results shown in Table 1.

TABLE 1
ACE inhibitory activity of commercially available processed cheeses

|  | ACE inhibitory activity (units per gram) |
|---|---|
| 6P cheese manufactured by Company A | 212 |
| GP cheese (1) manufactured by Company B | 223 |
| 6P cheese (2) manufactured by Company B | 208 |
| Sliced cheese (1) manufactured by Company A | 199 |
| Sliced cheese (2) manufactured by Company A | 189 |
| Sliced cheese (1) manufactured by Company B | 156 |
| Sliced cheese (2) manufactured by Company B | 230 |
| Sliced cheese manufactured by Company C | 190 |

On the other hand, there are many attempts to utilize an enzyme such as protease and lipase in the processes for manufacturing milk products. For example, there are reports of Toyoda et al. (Utilization of enzyme in manufacturing milk products, Japanese Journal of Dairy and Food Science, Vol.. 34, No. 6, 1985, p. A221-A228), Nozaki (Development and utilization of natural flavor from the viewpoint of new technology and raw materials, Food and Development, Vol. 21, No. 12, 1986, p. 38-41), Kanizawa (Utilization of enzyme flavor, New Food Industry, Vol. 26, No. 11, 1984, p. 37-41), and Judie D. Dziezak et al. (Biotechnology and Flavor Development: Enzyme Modification of Dairy Products, Food Technology, April 1986, p. 114-120). Although such enzyme utilization may produce various types of hypotensive peptide, the purpose of these reports is only to utilize an enzyme to shorten the ripening time of cheese or to provide cheese or butter with good flavor.

However, in Japan where cheese consumption is not as high as in Western countries, it can be predicted that it may be difficult to make daily consumption of a certain amount of cheese a custom. In addition, excessive consumption of cheese may adversely promote high blood pressure due to salt in cheese. In case of the of the abovementioned soft drinks for high blood pressure prevention, daily intake of that drink is sat up to 5000 units converted to the ACE inhibitory activity. In order to supply this amount by consuming commercially available cheese shown in Table 1, the daily target consumption is 22-32 g. This amount is far larger than the Japanese average consumption of approximately 5.5 g. From these situations, it can be said that it would be very difficult for the current consumption of processed cheese to contribute to the prevention of high blood pressure. In addition, if it is possible to achieve an intake of the ACE inhibitory activity from milk products other than cheese, for example, yogurt or milk beverage that may be easier to accept than cheese, it is considered that the salt intake can be reduced. It is known that milk products other than cheese, for example, cow's milk and yogurt, have ACE inhibitory activity, although the amount of contained activity is very small (see the abovementioned report by H. Meisel et al.).

Disclosure of the Invention

This invention is made considering these situations. The purpose of the invention is to provide various types of functional food including milk products excellent in an antihypertensive effect that can be consumed in the condition closer to natural food life.

That is to say, this invention relates to a process for producing functional foods of which angiotensin converting enzyme inhibitory activity is enhanced through a regular intake in such a degree that high pressure can be controlled within the normal blood pressure range, wherein enzyme-modified cheese is added to a product.

Specifically, the content of enzyme-modified cheese is 1.5% or less and add enzyme-modified cheese that does not substantially produce odor peculiar to enzyme-modified cheese.

In this invention, cheese modified by two types or more of protease, enzyme-modified cheese modified by enzyme other than lipase, and more preferably, enzyme-modified cheese of which raw material is cheese with the fat content of 20% or less and the protein content of 28% or more, are used. In addition, enzyme-modified cheese having angiotensin converting enzyme inhibitory activity of 9,000 units per gram or more is used.

The functional foods in the invention are provided as processed cheese, and as yogurt.

Functional foods provided as processed cheeses in the invention have angiotensin converting enzyme inhibitory activity of 350 units per gram or more, and natural cheese with angiotensin converting enzyme inhibitory activity of 420 units per gram or more as a raw material is used.

More preferably, the sodium content is 990 mg or less per 100 g of processed cheese, and the potassium content is between 80 mg and 150 mg per 100 g of processed cheese.

When the functional food provided is yogurt in the invention, the angiotensin converting enzyme inhibitory activity is 50 units per gram or more.

Best Mode for Carrying out the Invention

The purpose of this invention is to blend enzyme-modified cheese (EMC) to provide functional foods having enhanced angiotensin converting enzyme inhibitory activity to prevent high blood pressure through daily customary eating. The functional foods in the invention have a characteristic that enzyme-modified cheese is contained to enhance angiotensin converting enzyme inhibitory activity in a product in such a degree that high pressure can be substantially controlled within the normal blood pressure range through daily consumption. That is to say, the functional food according to the invention is food capable of standing for hypotensive function such as so-called food for specified health uses, of which consumption demonstrates a function to prevent high blood pressure and maintain blood pressure within the normal range. In order to demonstrate this function to achieve the aforementioned purpose, the specific target is determined to be 5,000 units or more per daily standard consumption of the product by using enzyme-modified cheese. In the invention, the ACE inhibitory activity is a value measured in the process described in Embodiments.

EMC is cheese that is obtained from green cheese, natural cheese ripened or being ripened or processed cheese to which various types of enzyme originated from microorganism or animal such as lipase, acid protease and neutral protease are added to promote ripening. The inventors completed this invention by noticing there is a possibility that a certain type of peptide has an antihypertensive effect stronger than that of normal cheese because some types of peptide has ACE inhibitory activity and protein of EMC is decomposed in the level higher than that of cheese. Table 2 shows measurement results of ACE inhibitory activity of commercially available EMC, demonstrating the EMC has inhibitory activity around ten times higher than the average ACE inhibitory activity (200-250 units per gram) of commercially available cheeses.

TABLE 2

ACE inhibitory activity of commercially available EMC

| Product | ACE inhibitory activity (units per gram) | Flavor of processed cheese to which EMC is added at a rate of 1.5% |
|---|---|---|
| Company A (1) | 2,450 | Too strong |
| Company A (2) | 2,508 | Allowable |
| Company A (3) | 1,912 | Good |

(continued)

ACE inhibitory activity of commercially available EMC

| Product | ACE inhibitory activity (units per gram) | Flavor of processed cheese to which EMC is added at a rate of 1.5% |
|---|---|---|
| Company B (1) | 2,253 | Too strong |
| Company B (2) | 4,863 | Too strong |
| Company B (3) | 6,751 | Too strong |
| Company B (4) | 2,930 | Too strong |

Originally, EMC has been used for providing flavor and taste in order to improve or enhance flavor of cheeses and other milk products, and is added when a large amount of raw material cheese with short ripening time (normally around three months) and light taste is used. Therefore, EMC with strong flavor as shown in Table 2 is generally required. However, for the purpose of the invention, it is preferable to select EMC with flavor as light as possible and ACE inhibitory activity as high as possible or to prepare such EMC for this purpose from the viewpoint that (1) it is preferable to increase the adding amount of EMC, (2) EMC is used with ripened raw material cheese, and (3) original flavor of food to which EMC is added is not adversely affected. Especially, since EMC produces strong odor even in a small amount, it is necessary to use EMC with ACE inhibitory activity as high as possible when preparation is made by mixing EMC in milk or soft drink. Therefore, the EMC used in the invention has inhibitory activity of 9,000 units per gram or more later.

In addition, since according to the invention EMC is added to various types of food, EMC excellent in not only ACE inhibitory activity but also in flavor is preferable. Relating this, combination of various types of protease enables to obtain EMC with a large amount of content of hypotensive peptides.

Protease is classified into endo- and exo-types, depending on the breaking portion of amino acid sequence. Endo-type protease cleaves proteins from the middle, and exo-type chews one by one from C-end or N-end of amino acid. Moreover, optimum pH, temperature, protein resolution, and breaking portion of amino acid vary depending on the types of protease. Thus a combination of two types or more, or preferably three types or more, of protease having different endo- or exo-type, protein resolution, optimum pH or temperature enables to produce a larger amount of hypotensive peptides. Usable protease and its combination have no special limitation, and commercially available protease can be properly combined. It is preferable to combine endo- and exo-types. For example, Protease N Amano (manufactured by Amano Enzyme Inc.), Protease S Amano (ditto), Newlase A (ditto), Umamizyme (ditto), Flavourzyme 500 L (manufactured by Novozyme Japan Co., Ltd.), Sumityme FP (manufactured by Shin Nihon Chemical, Co., Ltd.), Molsin F (manufactured by Kikkoman Corporation), Orientase 20A (HBI Co., Ltd.), and so on can be listed up as usable protease.

Enzyme treatment is performed by adding the abovementioned protease to raw material cheese and activating the protease in the condition appropriate for the protease being used. In actual treatment, two types or more of protease can be used simultaneously or each protease can be added one by one to repeat activation and deactivation.

It is well known that the obtained EMC contains many types of fatty acid; however, it is considered that fatty acid does not so much contribute to high blood pressure prevention, and EMC that produces only a small amount of fatty acid is preferable from the viewpoint of flavor. Therefore, mainly using cheese having the small content of fat as a raw material of EMC results in the protein content of EMC higher than that of normal natural cheese, enabling to increase the content of hypotensive peptide. And, EMC having weaker flavor can be obtained. Specifically, it is preferable to combine cheese having the normal content of fat and low fat cheese or skim cheese to use cheese with the fat content of 20% or less and the protein content of 28% or more. More preferably, it is desirable to use cheese with the fat content and protein content 15% or less and 30% or more, respectively. These values of both the fat content and protein content were measured in the process provided in the Food Sanitation Law.

In the invention, lipase is not used although that has been used in the conventional EMC manufacturing process to produce larger amount of short chain fatty acid as a flavor component. Thus production of strong flavor is restricted, leading to milder flavor, and it becomes easier to add EMC to processed cheese, cheese food, yogurt, and the other foods abovementioned. A combination of protease and use of cheese having the low content of fat and high content of protein can raises ACE inhibitory activity three to ten times higher than that of conventional EMC, and in addition, EMC having weak flavor can be obtained.

When EMC with ACE inhibitory activity of 2,500 units per gram that is considered to be of commercially available standard product is added, the calculated value of ACE inhibitory activity is 250 units per gram, which cannot be said too high in comparison with the commercially available processed cheese shown in Table 1. However, when as in accordance with the invention ACE inhibitory activity of EMC is 9,000 units per gram, the calculated value of ACE inhibitory activity of product-processed cheese is 347.5 units per gram, and when ACE inhibitory activity of EMC is 9,200 units per gram, the calculated value of ACE inhibitory activity is 350.5 units per gram. These values show that the daily intake target of ACE inhibitory activity of 5,000 units per gram can be sufficiently achieved by consuming cheese at a rate of 15 grams per day. It is recommended to make EMC content 2% or less, preferably 1.5% or less and more preferably 1.0% or less, although it depends on flavor of EMC (content of fatty acid) and its ACE inhibitory activity.

TABLE 3

|  | ACE inhibitory activity (units per gram) | Content (%) |
|---|---|---|
| Raw material natural cheese | 250 | 85.0 |
| EMC | 2,500 | 1.5 |
| Molten salt | - | 2.0 |
| Water | - | 11.5 |

In the invention, the required ACE inhibitory activity (specific activity) in food is determined by targeting 5,000 units per daily standard intake of the functional food. That is to say, the intake of peptide containing ACE inhibitory activity of 5,000 units is aimed by consuming a daily amount of the food. Needless to say, each of the foods has the different content of peptide (specific activity) per unit amount of the product. Now, the daily standard intake means the amount that is considered to be regularly consumed by the average Japanese per day and it is different from the actual average consumption as mentioned below. More specifically, the daily standard intake is 10-15 g for cheese, 180-200 ml for processed milk, 100-150 g for fermented milk, daily required amount (it depends on usage, for example, a guideline when drinking five times diluted liquid once a day may be 30-40 ml, and that when drinking five times diluted liquid twice a day may be 60-80 ml) for lactic acid bacteria beverage, about 100 g for ice cream, and about 500 ml for soft drink.

More generally speaking, the daily standard intake can also be said a standard amount that is considered to be daily consumed by the Japanese. For example, in case of cheeses, the average cheese consumption of the Japanese is approximately 5.5 g, which is too small in product size, even from the viewpoint of the balance of lactoprotein or calcium intake. On the other hand, although cheese of which the weight per piece is 20-25 g is commercially available, this amount is too large to be daily consumed by the average Japanese. Therefore even taking the increase of cheese intake in the future into consideration, daily intake is considered to be 10-15 g. Standard daily intake of processed milk is an amount of a milk bottle, 180-200 ml, and that of yogurt is considered to be an amount of a cup, namely about 100 g as an average. Since this standard is basically a guideline, that value should be determined depending on the characteristics of each food so as to achieve the daily intake of ACE inhibitory activity of 5,000 units.

Thus, the ACE inhibitory activity contained in a unit amount of product is determined. For example, when the standard intake of processed cheese is assumed to be 10-15 g, the required ACE inhibitory activity of processed cheese is 333-500 units per gram. In other words, it is necessary to take 350 units per gram or more, preferably 500 units per gram or more, and more preferably 700 units per gram from processed cheese. In other cheeses, the standard intake is adjusted to 350 units per gram or more, preferably 500 units per gram or more, and more preferably 700 units per gram in the same way of thinking as processed cheese.

Using raw material natural cheese having the value of 420 units per gram or more equivalent to 85% or more of total raw material cheese enables to obtain cheese with the aforementioned target value of 350 units per gram or more without using EMC. However, using EMC enables to obtain cheese having further higher ACE inhibitory activity. For example, referring to the data in Table 3, when adding EMC having ACE inhibitory activity of 9,000 units per gram to raw material natural cheese having ACE inhibitory activity of 420 units per gram at a rate of 1.5% to the product, the calculated value of ACE inhibitory activity of the product processed cheese is 492 units per gram, which is a value about two times higher than current commercially available cheese. Thus the consumption of cheese required to obtain a sufficient antihypertensive effect is about a half of the current consumption, namely about 10-12 g. From this way of thinking, it is desirable to use EMC having ACE inhibitory activity of 9,000 units per gram or more, preferably 12,000 units per gram or more, and more preferably 14,000 units per gram or more. The EMC having high ACE inhibitory activity like this can be easily obtained by applying the abovementioned two or more types of protease to process raw material cheese and further using cheese having the fat content of 20% or less and the protein content of 28% or more as raw material cheese.

In addition, EMC should substantially be used for adding flavor as abovementioned. Since excessive addition of EMC may sometimes produce undesirable flavor or taste without providing the original flavor of milk products, the excessive addition should be avoided. Therefore, in manufacturing cheeses, it is desirable to use raw material cheese with ACE inhibitory activity of 420 units per gram or more, preferably 500 units per gram or more, and more preferably 700 units per gram or more as mentioned above.

Moreover, in manufacturing processed cheeses, a combination of multiple types of cheese is often used as the raw material cheese. The purpose is to adjust flavor, adjust the degree of ripening, alleviate quality variation of each raw material cheese, and so on. Also in this invention, multiple types of raw material cheese can be combined to make

EP 1 570 744 B1

the ACE inhibitory activity of total raw material cheese 420 units per gram or more, preferably 500 units per gram or more, and more preferably 700 units per gram or more. The types of cheese to be combined have no special limitation. For example, cheddar cheeses, such as cheddar, Cheshire, Colby and Monterey jack; Gouda type cheeses, such as Gouda, Samso and Maribo; Edam cheeses; extremely hard cheeses, such as parmesan, Romano and Granapadano; Swiss cheeses, such as Emmentaler and Gruyeres; white mold cheeses, such as Camembert and Brie; blue mold cheeses, such as Stilton, Roquefort, gorgonzola and Dana blue; cheeses of which the surface is ripened by bacteria, such as limburger; unripened cheeses, such as cream cheese, mascarpone, cottage and mozzarella; and whey cheeses such as ricotta can be used.

An amount of hypotensive peptide in cheese is different in each cheese. It varies depending on the types of cheese and the ripening time. According to the data for commercially available raw material cheese measured by the inventors, an amount of peptide contained in unripened cheeses (fresh cheeses such as cottage cheese, cream cheese and quark, as well as unripened curd) is small because of the low degree of proteolysis, leading to a small antihypertensive effect. Not only that, it was found that the antihypertensive effect greatly varies depending on manufacturing methods (manufacturers) even if the type of cheese and the ripening time are the same in ripened cheeses. The ripening time, which is only a guideline, does not provide sufficient information for fully evaluating the strength of the antihypertensive effect. Thus, it is necessary to select a type of cheese after measuring the antihypertensive effect of each cheese. A part of the data of ACE inhibitory activity of natural cheese measured by the inventors is shown in Table 4. This table shows that cheddar cheese produced in New Zealand and Emmentaler cheese produced in Switzerland have a peculiarly large antihypertensive effect as examples. Therefore, in order to achieve the purpose of the invention, it is preferable to use cheddar cheese produced in New Zealand, Emmentaler cheese produced in Switzerland, or a combination of both, because these cheeses with a peculiarly large antihypertensive effect have ACE inhibitory activity of at least 420 units per gram. In addition, even when using these cheeses, it is preferable to use those having ACE inhibitory activity of 500 units per gram or more, and more preferably 700 units per gram or more.

TABLE 4

ACE inhibitory activity of raw material natural cheese

|  | Number of maturity months | ACE inhibitory activity (units per gram) |
|---|---|---|
| Domestic Gouda cheese | 1 | 151 |
| Domestic Gouda cheese | 6 | 244 |
| Gouda cheese produced in Netherlands | 5 | 229 |
| Gouda cheese produced in Netherlands | 8 | 227 |
| Gouda cheese produced in New Zealand | 3 | 149 |
| Gouda cheese produced in New Zealand | 6 | 173 |
| Cheddar cheese produced in New Zealand | 7 | 709 |
| Cheddar cheese produced in New Zealand | 17 | 1141 |
| Cheddar cheese produced in Canada | 6 | 236 |
| Cheddar cheese produced in Canada | 13 | 277 |
| Parmesan cheese produced in Australia | 12 | 100 |
| Parmesan cheese produced in Australia | 20 | 93 |
| Emmentaler cheese produced in Switzerland | 06 | 525 |

On the other hand, it is well known that sodium acts to raise blood pressure. Processed cheese contains sodium from salt (content of 1.1-1.5 wt% in the product) and molten salt(content of 2-2.5 wt% in the product) such as phosphate, and citrate. Therefore, reduction of sodium in processed cheese products can further increase an antihypertensive effect of hypotensive peptides. The sodium content of standard processed cheese is 1,100 mg per 100 g of cheese (according to the Japan Food Content Table Ver. 5). In addition, cheese containing sodium at 980 mg or less per 100 g is classified as low-salt sodium food that is preferable for preventing high blood pressure. Therefore, the sodium content of processed cheese product should be adjusted to 990 mg per 100 g of cheese.

Although a smaller amount of sodium content is better, a certain amount of sodium is necessary from the viewpoint of flavor and protection from bacteria. Moreover, since molten salt is also an essential additive for emulsification of processed cheeses, it is difficult to stop using molten salt. Therefore, it is preferable to properly combine unsalted natural cheese and low-salt natural cheese as a part of the raw material for processed cheese to reduce the sodium content. Further, the molten salt is sometimes called an emulsifier.

Sodium from molten salt can be reduced by substituting potassium salt for a part of the sodium. Since potassium functions to lower blood pressure, substitution by potassium salt is an effective method for achieving the purpose of the invention. However, since potassium salt has a peculiar bitter taste, the potassium content is limited to 150 mg or less per 100 g of cheese. In this connection, since the potassium content of normal processed cheese is around 60 mg per 100 g of cheese, it is necessary to increase the amount of potassium in order to reduce the sodium content. Thus, in this invention, it is preferable to make the potassium content between 80 mg per 100 g of cheese and 150 mg per 100 g of cheese.

Processed cheese that was taken as a main example in the above description originally has a large amount of peptides with ACE inhibitory activity, while daily consumption is not enough to achieve the desired target. On the other hand, milk products other than processed cheeses, such as yogurt, ice cream and milk beverage, has a small amount of peptides with ACE inhibitory activity, but daily consumption is relatively large. Thus, it is possible to realize the daily intake of ACE inhibitory activity of 5,000 units by adding a small amount of EMC. For example, if EMC with the typical content of ACE inhibitory activity (2,500 units per gram) shown in table 3 is used, daily consumption of EMC of 2 g can achieve the purpose of this invention. When, for example, consumption of milk beverage is 200 ml, the above purpose can sufficiently be accomplished by adding EMC at a rate of 1 wt%, and when consumption of yogurt is 100 ml, addition at a rate of 2 wt% is enough.

However, the addition of EMC in this amount often lowers a commercial value of products because odor of EMC is too strong. Therefore, taking the odor of EMC into consideration, it is considered to be preferable to make EMC content in yogurt 0.25-0.5 wt%, and at most 1 wt%. Thus even in milk products other than processed cheese, namely yogurt, it is desirable to use EMC having ACE inhibitory activity of 9,000 units per gram or more, preferably 12,000 units per gram or more, and more preferably 14,000 units per gram or more. It is a matter of course that the content of EMC to be mixed in milk products and ACE inhibitory activity of EMC to be used are substantially guidelines, and that they are finally determined considering odor, taste, and the specified consumption of milk product. In these milk products, EMC can be added in any process during manufacturing, and EMC can be added to raw material milk to ferment the milk to prepare lactic acid bacteria beverage and fermented milk.

Also in the other foods, types of EMC (for example, ACE inhibitory activity and raw material EMC) to be mixed and an amount of EMC to be added can be properly determined considering odor, taste and daily consumption of the food in the same way as the milk products.

DESCRIPTION OF THE PREFERRED EMBODIMENTS

Hereafter the invention is specifically explained by describing the embodiments. However, the invention is not restricted by the following embodiments:

(EMBODIMENT 1)

First, EMC with high ACE inhibitory activity was manufactured using skim cheese, endo-type protease (enzymes A and C) and exo-type protease (enzyme B).

[Composition]

| | |
|---|---|
| Skim cheese produced in Australia (ripening time of five months) with fat content of 9.0% and protein content of 35.0% | 3.0 kg |
| Mozzarella cheese produced in Australia (ripening time of five months) with fat content of 22.1% and protein content of 25.9% | 2.0 kg |
| Skim cheese produced in Denmark (ripening time of four months) | 5.0 kg |
| Starter culture solution (lactic acid bacteria A) | 0.45 kg |
| Starter culture solution (lactic acid bacteria B) | 0.45 kg |
| Enzyme A (Protease N Amano) | 0.03 kg |
| Enzyme B (Flavourzyme) | 0.015 kg |
| Enzyme C (Umamizyme) | 0.017 kg |
| Sodium chloride | 0.17 kg |
| Citric acid | 0.35 kg |
| Sodium hydroxide (8N) | 0.05 L |
| Water | 2.93 kg |

[Process]

Water and raw material cheese shredded in a meat chopper were blended and agitated. After sterilizing at 85°C for 10 minutes, two types of starter culture solution of lactic acid bacteria that had been cultured in the culture medium of skimmilk powder in advance were added and agitated, and enzyme A was further added and mixed. The solution was agitated and blended at 34°C for 48 hours to allow enzyme A to activate. In order to lower pH, citric acid was added to prepare solution having pH of 4.1, and then enzymes B and C were added and blended. The solution was further agitated and blended at 34°C for six days to allow the enzymes to activate. After eight days from the start of enzyme A activation, decomposition was ended, aqueous solution of sodium hydroxide was added to adjust pH to 5.0, and the enzymes were deactivated by heating the solution at 85°C for 10 minutes to obtain EMC. ACE inhibitory activity of this EMC was 23,396 units per gram, providing good flavor without producing odor peculiar to EMC. By the way, (A) mixture of *L. lactis subsp. lactis, L. cremaris* and *L. diacetylactis* and (B) *L. lactis subsp. lactis* were used as lactic acid bacteria.

[Measurement of ACE inhibitory activity]

Pretreatment of samples was performed as follows:

Filtered 800 ml of pure water is added to 1 g of EMC or cheese and agitated by a mixer for five minutes. Then the material is subjected to centrifugal separation at 7,000 rpm for 10 minutes to split the water layer and remove siltation and the upper oily layer. The water layer is filtered to obtain the sample liquid.

Measurement of ACE inhibitory activity is performed as follows:

Sample liquid is neutralized using 1 N of sodium hydroxide. The neutralized 0.04 ml of sample liquid is mixed with 0.1 ml of enzyme liquid (Angiotensin Converting Enzyme: 2 units per milliliter) to heat the mixture to 37°C. Then the 0.1 ml matrix layer (Hippuryl-His-Leu; N-Benzoyl-Gly-His-Leu) is added and sufficiently agitated. The liquid is kept at 37°C for 60 minutes to allow a reaction. After the reaction, 0.13 ml of 1 N hydrochloric acid is added and sufficiently agitated to stop the reaction. Then 0.85 ml of ethyl acetate is added, shaken for one minute, and subjected to centrifugal separation at 3000 rpm for 10 minutes. Supernatant (0.7 ml) is collected to remove the solvent by a centrifugal evaporator (for about 30 minutes). Then, 0.5 ml of distilled water is added to this to dissolve residual substances to measure absorbance at a wave length of 228 nm. ACE inhibitory activity (units per gram) is calculated using the following Equation 1. In the equation, $A$ is absorbance of the control when an enzyme is used, $B$ is that of the control when no enzyme is used, $C$ is that of the sample liquid when an enzyme is used, and $D$ is that of the sample liquid when no enzyme is used. Water was used as a control instead of the sample liquid.

Equation 1 Inhibitory activity

$$\text{Inhibitory activity(unit/g)} = \frac{(A-B)-(C-D)}{(A-B)} \times \frac{100}{50} \times \frac{1}{0.04} \times 800$$

(EMBODIMENT 2)

In the same way as above, EMC having high ACE inhibitory activity was manufactured using skim cheese, endo-type protease (enzymes A and C) and exo-type protease (enzyme B). The process is the same as Embodiment 1.

[Composition]

| | |
|---|---|
| Skim cheese produced in Denmark (ripening time of four months) with fat content of 5.0% and protein content of 39.7% | 5.0 kg |
| Starter culture solution (lactic acid bacteria A) | 0.45 kg |

(continued)

| Starter culture solution (lactic acid bacteria B) | 0.45 kg |
|---|---|
| Enzyme A (Protease S Amano) | 0.03 kg |
| Enzyme B (Newlase A) | 0.015 kg |
| Enzyme C (Umamizyme) | 0.017 kg |
| Sodium chloride | 0.17 kg |
| Citric acid | 0.35 kg |
| Sodium hydroxide (8N) | 0.05 L |
| Water | 2.93 kg |

It was found that the obtained EMC has ACE inhibitory activity of 19,912 units per gram, having good flavor without producing odor peculiar to EMC.

(EMBODIMENT 3)

In the same way as above, EMC having high ACE inhibitory activity was manufactured using skim cheese, endo-type protease (enzyme A) and exo-type protease (enzyme B).

[Composition]

| Skim cheese produced in Denmark (ripening time of four months) with fat content of 5.0% and protein content of 39.7% | 5.0 kg |
|---|---|
| Starter culture solution (lactic acid bacteria A) | 0.45 kg |
| Starter culture solution (lactic acid bacteria B) | 0.45 kg |
| Enzyme A (Protease N Amano) | 0.3 kg |
| Enzyme B (Sumityme FP) | 0.3 kg |
| Sodium chloride | 0.17 kg |
| Citric acid | 0.35 kg |
| Sodium hydroxide (8 N) | 0.05 L |
| Water | 2.93 kg |

[Process]

Water and raw material cheese shredded in a meat chopper were blended and agitated. After sterilizing at 85°C for 10 minutes, two types of starter culture solution of lactic acid bacteria that had been cultured in the culture medium of skimmilk powder in advance were added and agitated, and enzyme A was further added and mixed. The solution was agitated and blended at 34°C for 48 hours to allow enzyme A to activate. In order to lower pH, citric acid was added to prepare solution having pH of 4.1, and then enzymes B was added and blended. The solution was further agitated and blended at 34°C for six days to allow the enzyme to activate. After eight days from the start of enzyme A activation, decomposition was ended, aqueous solution of sodium hydroxide was added to adjust pH to 5.0, and the enzymes were deactivated by heating the solution at 85°C for 10 minutes to obtain EMC. ACE inhibitory activity of this EMC was 19,528 units per gram, providing good flavor without producing odor peculiar to EMC.

(EMBODIMENT 5)

EMC having ACE inhibitory activity of approximately 19,000 units per gram was added to cheddar cheese produced in New Zealand (ripening time of 12 months and ACE inhibitory activity of 940 units per gram) as a raw material to prepare processed cheese in the following process. The composition is as follows:

[Composition]

| Cheddar cheese produced in New Zealand | 8.0 kg |
|---|---|
| EMC | 0.05 kg |
| Molten salt (sodium tripolyphosphate) | 0.2 kg |
| Water (including steam for heating) | 1.75 kg |

[Process]

Raw material cheese was coarsely shredded in advance using a meat chopper. All the raw materials were fed to a kettle type kneader with a volume of 20 liters (however the amount of water does not include that of steam for heating), and were mixed at a speed of 120 rpm while blowing in steam for about 10 minutes to heat the materials to 85°C. Two hundred grams each of fluid molten cheese were sampled into containers, which were tightly closed to cool in a refrigerator at 5°C for one night. This processed cheese was found to have good flavor and texture without producing odor peculiar to EMC. In addition, the measured value of ACE inhibitory activity of 660 units per gram demonstrated that processed cheese having ACE inhibitory activity sufficiently higher than commercially available processed cheese could be manufactured.

(EMBODIMENT 6)

EMC having ACE inhibitory activity of approximately 21,000 units per gram was added to Gouda cheese produced in New Zealand (ripening time of six months and ACE inhibitory activity of 170 units per gram) as a raw material to prepare processed cheese. The process is the same as Embodiment 5.

[Composition]

| Gouda cheese produced in New Zealand | 8.5 kg |
|---|---|
| EMC | 0.1 kg |
| Molten salt (sodium polyphosphate) | 0.1 kg |
| Molten salt (disodium hydrogen phosphate) | 0.1 kg |
| Water (including steam for heating) | 1.2 kg |

This processed cheese was found to have good flavor and texture without producing odor peculiar to EMC. In addition, the measured value of ACE inhibitory activity of 370 units per gram demonstrated that processed cheese with ACE inhibitory activity sufficiently higher than commercially available processed cheese could be manufactured.

(EMBODIMENT 7)

EMC having ACE inhibitory activity of 14,000 units per gram was added to Gouda cheese produced domestically (ripening time of six months and ACE inhibitory activity of 240 units per gram) as a raw material to prepare processed cheese. The process is the same as Embodiment 5.

[Composition]

| Gouda cheese produced domestically | 6.5 kg |
|---|---|
| EMC | 1.5 kg |
| Molten salt (sodium polyphosphate) | 0.15 kg |
| Molten salt (sodium citrate) | 0.05 kg |
| Water (including steam for heating) | 1.2 kg |

This processed cheese was found to have good flavor and texture without producing odor peculiar to EMC. In addition, the measured value of ACE inhibitory activity of 360 units per gram demonstrated that processed cheese having ACE inhibitory activity sufficiently higher than commercially available processed cheese could be manufactured.

(EMBODIMENT 8)

EMC having ACE inhibitory activity of 18,000 units per gram was added to cheddar cheese produced in New Zealand (ripening time of seven months, ACE inhibitory activity of 709 units per gram, and salt content of 2 wt%) as a raw material to prepare processed cheese. The process is the same as Embodiment 5.

[Composition]

| | |
|---|---|
| Cheddar cheese produced in New Zealand | 8.0 kg |
| EMC | 0.1 kg |
| Molten salt (sodium polyphosphate) | 0.15 kg |
| Molten salt (potassium polyphosphate) | 0.05 kg |
| Water (including steam for heating) | 1.7 kg |

This processed cheese was found to have good flavor and texture without producing odor peculiar to EMC. In addition, the measured value of ACE inhibitory activity of 750 units per gram demonstrated that processed cheese having ACE inhibitory activity sufficiently higher than commercially available processed cheese could be manufactured. This processed cheese has the sodium content of 860 mg% and the potassium content of 120 mg%.

(EMBODIMENT 9)

EMC having ACE inhibitory activity of 18,000 units per gram was added to cheddar cheese produced in New Zealand (ripening time of 17 months, ACE inhibitory activity of 1141 units per gram, and salt content of 2.0 wt%) and low-salt cheddar cheese produced in New Zealand (ripening time of 0.5 months ACE inhibitory activity of 140 units per gram, and salt content of 0.5 wt%) for reducing saltiness as raw materials to prepare processed cheese. The process is the same as Embodiment 5.

[Composition]

| | |
|---|---|
| Cheddar cheese produced in New Zealand | 6.5 kg |
| Low-salt cheddar cheese produced in New Zealand | 1.5 kg |
| EMC | 0.1 kg |
| Molten salt (sodium polyphosphate) | 0.2 kg |
| Water (including steam for heating) | 1.7 kg |

This processed cheese was found to have good flavor and texture without producing odor peculiar to EMC. In addition, the measured value of ACE inhibitory activity of 650 units per gram demonstrated that processed cheese having ACE inhibitory activity sufficiently higher than commercially available processed cheese could be manufactured. This processed cheese has the sodium content of 950 mg%.

(EMBODIMENT 10)

EMC having ACE inhibitory activity of 15,000 units per gram was added to cheddar cheese produced in New Zealand (ripening time of seven months, ACE inhibitory activity of 709 units per gram, and salt content of 2.0 wt%) and Emmentaler cheese produced in Switzerland (ripening time of nine months ACE inhibitory activity of 525 units per gram, and salt content of 1.8 wt%) as raw materials to prepare cheese spread. The process is the same as Embodiment 5.

[Composition]

| | |
|---|---|
| Cheddar cheese produced in New Zealand | 4.0 kg |
| Emmentaler cheese produced in Switzerland | 2.0 kg |
| Vegetable oil (rapeseed oil) | 0.1 kg |
| EMC | 1.1 kg |
| Molten salt (sodium polyphosphate) | 0.15 kg |
| Molten salt (potassium polyphosphate) | 0.05 kg |
| Water (including steam for heating) | 2.7 kg |

This processed cheese was found to have good flavor and texture without producing odor peculiar to EMC. In addition, the measured value of ACE inhibitory activity of 550 units per gram demonstrated that processed cheese having ACE inhibitory activity sufficiently higher than commercially available processed cheese could be manufactured. This processed cheese has the sodium content of 850 mg% and the potassium content of 120 mg%.

(EMBODIMENT 11)

Plain yogurt to which EMC is added was prepared as follows: The culture medium of 10 wt% skimmilk powder was inoculated with *L. bulgaricus* JCM 1002T and *S. thermophilus* ATCC 19258 at a rate of 1 wt% each, and cultured at 37°C for 15 hours to prepare bulk starter. Then the specified amount of EMC having ACE inhibitory activity of 15,000 units per gram, 80 wt% of milk, 2 wt% of skimmilk powder, and the specified amount of water were blended as shown in Table 5, and the mixture was homogenized at 65°C at a pressure of 150 kg/cm$^2$. This mixture was sterilized at 95°C for five minutes and cooled down to 43°C. This material was inoculated with the aforementioned bulk starter at a rate of 2 wt%. Sterilized containers were filled with the material at an amount of 100 g each in a sterilized condition. The materials were fermented at 43°C for four hours and cooled at 5°C after completing the fermentation. The test results (ACE inhibitory activity was measured according to the abovementioned process) of this yogurt are shown in Table 5.

TABLE 5

| | Amount of EMC added (%) | Water (%) | ACE inhibitory activity (units per gram) | Acidity (%) | pH | Flavor |
|---|---|---|---|---|---|---|
| Embodiment 1 | 0.50 | 15.5 | 75 | 0.86 | 4.60 | Good |
| Embodiment 2 | 1.00 | 15.0 | 150 | 0:88 | 4.58 | Good but cheese-like flavor slightly produced |
| Comparison example 1 | 2.00 | 14.0 | 300 | 0.91 | 4.65 | Too strong cheese flavor and peculiar odor produced |

In this yogurt also, it was recognized that ACE inhibitory activity could be sufficiently enhanced, and the consumption of 100 ml of this yogurt enables the intake of 5,000 units of ACE inhibitory activity sufficiently.

This invention provides processed cheese and yogurt having an antihypertensive effect far higher than that of commercially available products, and enables to naturally control blood pressure by eating the milk products daily. Especially processed cheese of which the sodium content is adjusted to the low level greatly contributes to controlling the sodium salt intake and blood pressure. Enzyme-modified cheese (EMC) with high ACE inhibitory activity provides yogurt as a milk product that can be consumed in the same way as conventional milk products by reducing its content in milk products to restrict odor peculiar to EMC.

**Claims**

1. A process for producing functional food wherein enzyme-modified cheese having angiotensin converting enzyme inhibitory activity of 9,000 units per gram or more obtained by a treatment with two types or more of proteases or an enzymatic treatment other than lipase treatment is added to natural cheese having angiotensin converting enzyme inhibitor activity of at least 420 units per gram in an amount of 1.5 % by weight or less to obtain processed cheese with angiotensin converting enzyme inhibitory activity of 350 units per gram or more, or

wherein enzyme-modified cheese having angiotensin converting enzyme inhibitory activity of 9,000 units per gram or more obtained by a treatment with two types or more of proteases or an enzymatic treatment other than lipase treatment is added to yogurt in an amount of 1.5 % by weight or less to obtain yogurt with angiotensin converting enzyme inhibitory activity of 50 units per gram or more.

2.  A process for producing function food according to Claim 1 wherein enzyme-modified cheese made from raw material cheese with a fat content of 20% or less and a protein content of 28% or more is used.

3.  A process for producing functional food according to Claims 1 or 2 wherein at least one type of natural cheese having angiotensin converting enzyme inhibitory activity of 420 units per gram or more is used in addition to said enzyme-modified cheese to produce said processed cheese.

4.  A process for producing functional food according to Claim 3 wherein low-salt or unsalted natural cheese is used as said raw material natural cheese and the sodium content of the obtained processed cheese is made 990 mg or less per 100 g of processed cheese.

5.  A process for producing functional food according to one of Claims 3 or 4 wherein potassium salt is used as raw material molten salt and the sodium content of the obtained processed cheese is 990 mg or less per 100 g of processed cheese.

6.  A process for producing functional food according to one of Claims 3 through 5 wherein the potassium content of the obtained processed cheese is between 80 mg and 150 mg per 100 g of processed cheese.


**Patentansprüche**

1.  Verfahren zur Herstellung funktioneller Lebensmittel, wobei Enzym-modifizierter Käse mit Angiotensin-umwandelndes-Enzym Inhibitor-Aktivität von mindestens 9.000 Einheiten pro Gramm, der durch eine Behandlung mit mindestens zwei Arten von Proteasen oder eine andere enzymatische Behandlung als Lipase-Behandlung erhalten wird, zu natürlichem Käse mit Angiotensin-umwandelndes-Enzym Inhibitor-Aktivität von mindestens 420 Einheiten pro Gramm in einer Menge von maximal 1,5 Gewichts-% hinzugegeben wird, um Schmelzkäse mit Angiotensin-umwandelndes-Enzym Inhibitor-Aktivität von mindestens 350 Einheiten pro Gramm zu erhalten, oder
    wobei Enzym-modifizierter Käse mit Angiotensin-umwandelndes-Enzym Inhibitor-Aktivität von mindestens 9.000 Einheiten pro Gramm, der durch eine Behandlung mit mindestens zwei Arten von Proteasen oder eine andere enzymatische Behandlung als Lipase-Behandlung erhalten wird, zu Joghurt in einer Menge von maximal 1,5 Gewichts-% hinzugegeben wird, um Joghurt mit Angiotensin-umwandelndes-Enzym Inhibitor-Aktivität von mindestens 50 Einheiten pro Gramm zu erhalten.

2.  Verfahren zur Herstellung funktioneller Lebensmittel nach Anspruch 1, wobei Enzym-modifizierter Käse verwendet wird, der aus Rohmaterialkäse mit einem Fettgehalt von maximal 20 % und einem Proteingehalt von mindestens 28 % hergestellt ist.

3.  Verfahren zur Herstellung funktioneller Lebensmittel nach den Ansprüchen 1 oder 2, wobei mindestens eine Art von natürlichem Käse mit Angiotensin-umwandelndes-Enzym Inhibitor-Aktivität von mindestens 420 Einheiten pro Gramm zusätzlich zu dem Enzym-modifizierten Käse verwendet wird, um den Schmelzkäse herzustellen.

4.  Verfahren zur Herstellung funktioneller Lebensmittel nach Anspruch 3, wobei salzarmer oder salzfreier natürlicher Käse als der natürliche Rohmaterialkäse verwendet wird und der Natriumgehalt des erhaltenen Schmelzkäses auf maximal 990 mg pro 100 g Schmelzkäse eingestellt wird.

5.  Verfahren zur Herstellung funktioneller Lebensmittel nach einem der Ansprüche 3 oder 4, wobei Kaliumsalz als geschmolzenes Rohmaterialsalz verwendet wird und der Natriumgehalt des erhaltenen Schmelzkäses auf maximal 990 mg pro 100 g Schmelzkäse eingestellt wird.

6.  Verfahren zur Herstellung funktioneller Lebensmittel nach einem der Ansprüche 3 bis 5, wobei der Kaliumgehalt des erhaltenen Schmelzkäses zwischen 80 mg und 150 mg pro 100 g Schmelzkäse beträgt.

**Revendications**

1.  Procédé de production de produit alimentaire fonctionnel dans lequel du fromage à enzyme modifiée ayant une activité inhibitrice de l'enzyme convertissant l'angiotensine de 9000 unités par gramme ou plus obtenu par un traitement avec deux types ou plus de protéases ou un traitement enzymatique différent du traitement à la lipase est ajouté au fromage naturel ayant une activité inhibitrice de l'enzyme convertissant l'angiotensine d'au moins 420 unités par gramme en une quantité de 1,5 % en poids ou moins pour obtenir du fromage fondu avec une activité inhibitrice d'enzyme convertissant l'angiotensine de 350 unités par gramme ou plus, ou
    dans lequel du fromage à enzyme modifiée ayant une activité inhibitrice de l'enzyme convertissant l'angiotensine de 9000 unités par gramme ou plus obtenu par un traitement avec deux types ou plus de protéases ou un traitement enzymatique différent du traitement à la lipase est ajouté à du yaourt en une quantité de 1,5 % en poids ou moins pour obtenir du yaourt avec une activité inhibitrice d'enzyme convertissant l'angiotensine de 50 unités par gramme ou plus.

2.  Procédé de production de produit alimentaire fonctionnel selon la revendication 1 dans lequel le fromage à enzyme modifié formé à partir de fromage de matière brute avec une teneur en graisse de 20 % et une teneur en protéines de 28 % ou plus est utilisé.

3.  Procédé de production de produit alimentaire fonctionnel selon les revendications 1 ou 2 dans lequel au moins un type de fromage naturel ayant une activité inhibitrice de l'enzyme convertissant l'angiotensine de 420 unités par gramme ou plus est utilisé en plus dudit fromage à enzyme modifié pour produire ledit fromage fondu.

4.  Procédé de production de produit alimentaire fonctionnel selon la revendication 3 dans lequel du fromage à faible teneur en sel ou non salé est utilisé comme ledit fromage naturel de matière brute et la teneur en sodium du fromage fondu obtenu est formé de 990 mg ou moins par 100 g de fromage fondu.

5.  Procédé de production de produit alimentaire fonctionnel selon une des revendications 3 ou 4 dans lequel du sel de potassium est utilisé comme sel en fusion de matière brute et la teneur en sodium du fromage fondu obtenu est de 990 mg ou moins par 100 g de fromage fondu.

6.  Procédé de production de produit alimentaire fonctionnel selon une des revendications 3 à 5 dans lequel la teneur en potassium du fromage fondu obtenu est entre 80 et 150 mg par 100 g de fromage fondu.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. SAITO et al.** Isolation and Structural Analysis of Antihypertensive Peptides That Exist Naturally in Gouda Cheese. *Journal of Dairy Science, USA,* 2000, vol. 83 (7), 1434-1440
- **H. MEISEL et al.** ACE-inhibitory activities in milk products. *Milchwissenschaft,* 1997, vol. 52 (6), 307-311
- **T. ITO et al.** Angiotensin converting enzyme inhibitor in cheese. *Medicine and Biology,* 1987, vol. 115 (6), 375-377
- **TOYODA et al.** Utilization of enzyme in manufacturing milk products. *Japanese Journal of Dairy and Food Science,* 1985, vol. 34 (6), A221-A228
- **NOZAKI.** Development and utilization of natural flavor from the viewpoint of new technology and raw materials. *Food and Development,* 1986, vol. 21 (12), 38-41
- **KANIZAWA.** Utilization of enzyme flavor. *New Food Industry,* 1984, vol. 26 (11), 37-41
- **JUDIE D. DZIEZAK et al.** Biotechnology and Flavor Development: Enzyme Modification of Dairy Products. *Food Technology,* April 1986, 114-120